# EUROPEAN PATENT APPLICATION

(11) **EP 3 275 489 A1**
(43) Date of publication of application: **31.01.2018**
(21) Application number: 16772026.7
(22) Date of filing: 29.02.2016
(51) Int. Cl.: A61M 5/32, A61M 5/50

(54) **INJECTION NEEDLE ASSEMBLY AND DRUG INJECTION DEVICE**

(30) Priority: 27.03.2015 JP 2015065474
(71) Applicant: Terumo Kabushiki Kaisha, Tokyo 151-0072 (JP)
(72) Inventor: IWASE Yoichiro, Nakakoma-gun Yamanashi 409-3853 (JP)
(74) Representative: Casalonga
(86) International application number: PCT/JP2016/055998
(87) International publication number: WO 2016/158142

(57) **Abstract**

An injection needle assembly includes a needle tube, a needle hub configured to hold the needle tube, a protector, a biasing member, and a restriction mechanism. The restriction mechanism is configured to restrict the protector from moving from a first position to a second position again after the protector has moved from the first position to the second position and has further moved from the second position to the first position. Moreover, the biasing member is disposed close to a needle tip of the needle tube in an axial direction with respect to the restriction mechanism.

## Description

### Technical Field

The present invention relates to an injection needle assembly and a medicine injection apparatus used for puncturing a skin surface with a needle tip to inject a medicine to a skin upper layer portion.

### Background Art

In recent years, human infection with avian influenza has been reported, and there are growing concerns about many damages due to outbreak (pandemic) of infection from individual to individual. For this reason, prepandemic vaccines considered as highly effective for the avian influenza have been in stock all over the world. Moreover, study has been conducted on an increase in a vaccine manufacturing amount for prepandemic vaccine administration to many individuals.

The skin includes three portions of the epidermis, the dermis, and the subcutaneous tissue. The epidermis is a layer of about 50 to 200 µm from a skin surface, and the dermis is a layer of about 1.5 to 3.5 mm continuing from the epidermis. An influenza vaccine is generally administered in a hypodermic or intramuscular manner, and, therefore, is injected into a skin lower layer portion or deeper.

It has been reported that even when a dosage amount is reduced by administration of the influenza vaccine to a target region which is a skin upper layer portion where many immunocytes are present, an immunity acquiring ability equivalent to that of hypodermic or intramuscular administration is obtained. Thus, since the dosage amount can be reduced by administration of the prepandemic vaccine to the skin upper layer portion, the prepandemic vaccine can be administered to more individuals. Note that the skin upper layer portion means the epidermis and the dermis of the skin.

Various methods using a single needle, multiple needles, a patch, gas, etc. have been reported as the method for administering a medicine to the skin upper layer portion. Considering administration stability, reliability, and a manufacturing cost, the method using the single needle is most suitable as the method for administration to the skin upper layer portion. Since early times, a Mantoux's test has been known as the method for administering the vaccine to the skin upper layer portion by means of the single needle. The Mantoux's test is generally the method for administering a medicine of about 100 µl in such a manner that a needle having a short bevel needle tip with a size of 26 to 27 gauge is, from a diagonal direction of about 10 to 15°, inserted into the skin by about 2 to 5 mm.

However, it is difficult to perform the technique of the Mantoux's test for medicine administration, and for this reason, the success rate of the Mantoux's test depends on the skill of a doctor whose performs injection. There is a probability that a child especially moves during administration, and for this reason, it is difficult to administer the influenza vaccine by the Mantoux's test. Thus, development of a device configured to easily administer a vaccine to the skin upper layer portion has been demanded.

Patent Literature 1 describes an injection apparatus for the skin upper layer portion, the injection apparatus being configured such that a limiter having a skin contact surface is connected to a needle hub. The limiter described in Patent Literature 1 is provided at the periphery of a needle tube, and a clearance is formed between the limiter and the needle tube. The length (the protruding length) of the needle tube protruding from the skin contact surface of the limiter is defined as 0.5 to 3.0 mm so that a medicine can be administered to under the skin.

### Citation List

### Patent Literature

Patent Literature 1: JP 2001-137343 A

### Summary of Invention

### Technical Problem

However, in the injection apparatus described in Patent Literature 1, a needle tip end portion of the needle tube is kept protruding from the skin contact surface of the limiter, leading to a problem that there is a probability that a user is accidentally punctured with a needle tip of the needle tube after medicine administration or upon disposal of the medicine injection apparatus.

In view of the above-described problem, the present invention is intended to provide an injection needle assembly and a medicine injection apparatus configured to prevent accidental puncturing of a user with a needle tip of a needle tube after medicine administration or upon disposal.

### Solution to Problem

For solving the above-described problem and accomplishing the above-described purpose of the present invention, the injection needle assembly of the present invention includes a needle tube having a needle tip with which a biological body is able to be punctured, a needle hub configured to hold the needle tube, a protector, a biasing member, and a restriction mechanism. The protector is movable between a first position at which the needle tip of the needle tube is covered and a second position at which the needle tip of the needle tube is exposed. The biasing member is configured to bias the protector toward the needle tip of the needle tube along an axial direction of the needle tube. The restriction mechanism is configured to restrict the protector from moving from the first position to the second position again after the protector has moved from the first position to the second position and has further moved from the second position to the first position. The biasing member is disposed close to the needle tip of the needle tube in the axial direction with respect to the restriction mechanism.

Moreover, the medicine injection apparatus of the present invention includes an injection needle assembly and a syringe detachably attached to the injection needle assembly. The above-described injection needle assembly is used as the injection needle assembly.

### Advantageous Effects of Invention

According to the injection needle assembly and the medicine injection apparatus of the present invention, puncturing with the needle tip of the needle tube after use despite user's intension can be prevented.

### Brief Description of Drawings

Fig. 1 is a perspective view of a medicine injection apparatus of a first embodiment of the present invention.
Fig. 2 is a sectional view of the medicine injection apparatus of the first embodiment of the present invention.
Fig. 3 is a sectional view of the medicine injection apparatus of the first embodiment of the present invention in a state during puncturing.
Fig. 4 is a sectional view of the medicine injection apparatus of the first embodiment of the present invention in a state after puncturing.
Fig. 5 is a sectional view of a medicine injection apparatus of a second embodiment of the present invention.
Fig. 6 is a sectional view of the medicine injection apparatus of the second embodiment of the present invention in a state during puncturing.
Fig. 7 is a sectional view of the medicine injection apparatus of the second embodiment of the present invention in a state after puncturing.

### Description of Embodiments

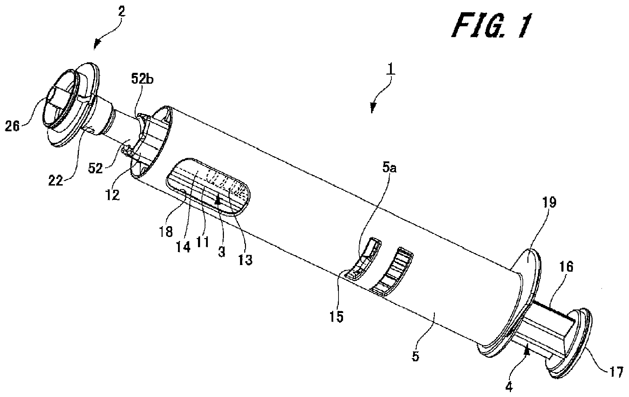

Embodiments of an injection needle assembly and a medicine injection apparatus according to the present invention will be described below with reference to Figs. 1 to 7. Note that in each drawing, the same reference numerals are used to represent common members. Moreover, the present invention is not limited to the embodiments described below.

Note that description will be made in the following order:
1. First Embodiment
1-1. Configuration Examples of Injection Needle Assembly and Medicine Injection Apparatus
1-2. Method for Using Medicine Injection Apparatus
2. Second Embodiment

### <1. First Embodiment>

### 1-1. Configuration Examples of Injection Needle Assembly and Medicine Injection Apparatus

First, an injection needle assembly and a medicine injection apparatus according to the first embodiment (hereinafter referred to as a "present embodiment") of the present invention will be described with reference to Figs. 1 to 2.

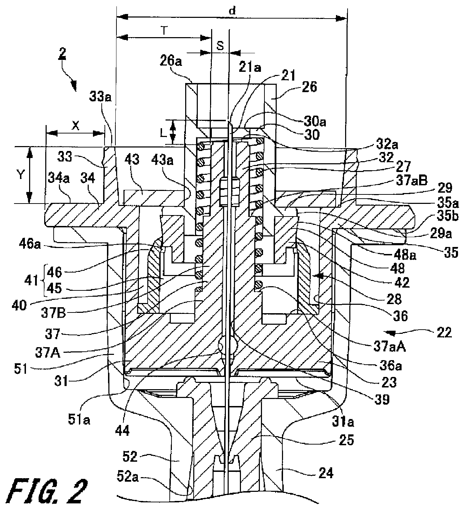

Fig. 1 is a perspective view of the medicine injection apparatus of the present embodiment, and Fig. 2 is a sectional view of the injection needle assembly of the present embodiment.

The medicine injection apparatus 1 is used to puncture a skin surface with a needle tip to inject a medicine into a skin upper layer portion. As illustrated in Fig. 1, the medicine injection apparatus 1 has the injection needle assembly 2, a syringe 3 detachably attached to the injection needle assembly 2, a pusher member 4, and a syringe holder 5 configured to hold the syringe 3.

### [Syringe]

The syringe 3 is a pre-filled syringe filled with the medicine in advance. The syringe 3 has a syringe body 11, a discharge portion formed at one end portion of the syringe body 11 in an axial direction, a lock mechanism 12 provided at the discharge portion, and a gasket 13.

The syringe body 11 is formed in a substantially hollow cylindrical shape. Moreover, the gasket 13 is slidably disposed in a cylinder hole of the syringe body 11. The gasket 13 is formed in a substantially circular columnar shape, and in a liquid-tight manner, closely contacts an inner peripheral surface of the cylinder hole of the syringe body 11. Moreover, the gasket 13 divides an inner space of the syringe body 11 into two spaces. The space on a discharge portion side with respect to the gasket 13 in the syringe body 11 serves as a liquid chamber 14 filled with the medicine. On the other hand, a plunger body 16 of the pusher member 4 as described later is inserted into the space on the other side with respect to the gasket 13 in the syringe body 11.

The material of the gasket 13 is, but not limited to, preferably an elastic material for realizing favorable liquid tightness with the syringe body 11. The elastic material includes, for example, various rubber materials such as natural rubber, isobutylene rubber, and silicone rubber, various thermoplastic elastomers such as olefin-based elastomer and styrene-based elastomer, and a mixture thereof.

The outer and inner diameters of the syringe body 11 are, as necessary, set according to a use application of the medicine injection apparatus 1 and the volume of medicine housed in the liquid chamber 14. For example, in a case where the volume of medicine housed using a general-purpose high-speed filling machine is 0.5 mL, the inner diameter of the syringe body 11 is preferably set to 4.4 to 5.0 mm, and the outer diameter of the syringe body 11 is preferably set to 6.5 to 8.4 mm. Moreover, in the case of a volume of 1 mL, the inner diameter of the syringe body 11 is preferably set to 6.1 to 9.0 mm, and the outer diameter of the syringe body 11 is preferably set to 7.9 to 12.5 mm.

For example, the medicine includes, but not limited to, various vaccines for preventing various types of infection such as influenza. Note that other medicines than the vaccines include, for example, sugar injection solutions such as glucose, electrolyte correction injection solutions such as sodium chloride or potassium lactate, vitamin supplements, antibiotic injection solutions, contrasts, steroids, protein degradation enzyme inhibitors, fatemulsions, anticancer drugs, anesthetics, heparin calcium, and antibody drugs.

The other end portion of the syringe body 11 in the axial direction is provided with a flange portion 15. The flange portion 15 is locked by a lock portion 5a provided at the syringe holder 5 as described later. Moreover, the one end portion of the syringe body 11 in the axial direction is provided continuously to the not-shown discharge portion.

The discharge portion is formed in a substantially cylindrical shape coaxial with the syringe body 11. Moreover, a cylinder hole of the discharge portion communicates with the cylinder hole of the syringe body 11. The discharge portion is formed in such a tapered shape that the diameter thereof is continuously decreased toward the one end portion in the axial direction. When the injection needle assembly 2 is attached to the syringe 3, a tip end portion of the discharge portion contacts, in a liquid-tight manner, an end surface of an elastic member 25 of the injection needle assembly 2 as described later.

The discharge portion is provided with the lock mechanism 12. The lock mechanism 12 is a lure lock portion as an example of a fixing mechanism. The lock mechanism 12 is formed in a tubular shape coaxially surrounding the discharge portion. Moreover, the lock mechanism 12 is formed with a circular inner periphery and a hexagonal outer periphery. An inner peripheral surface of the lock mechanism 12 is provided with an internal thread portion. The internal thread portion is formed to threadedly engage with an external thread portion 52b provided at the injection needle assembly 2.

The material of the syringe body 11 includes, for example, various resins such as polyvinyl chloride, polyethylene, polypropylene, cyclic polyolefin, polystyrene, poly-(4-methylpentene-1), polycarbonate, acrylic resin, acrylonitrile-butadiene-styrene copolymer, polyester such as polyethylene terephthalate, butadiene-styrene copolymer, and polyamide (e.g., nylon 6, nylon 6.6, nylon 6.10, and nylon 12). Of these materials, resins such as polypropylene, cyclic polyolefin, polyester, and poly-(4-methylpentene-1) are preferably used in terms of easy molding. Note that the material of the syringe body 11 is preferably substantially transparent for ensuring inner visibility.

Note that in the present embodiment, the example where the pre-filled syringe filled with the medicine in advance is applied as the syringe 3 has been described, but the present invention is not limited to this example. A syringe with a syringe body not filled with the medicine in advance may be applied.

### [Pusher Member]

The pusher member 4 has the plunger body 16 and an operation portion 17 configured to operate the plunger body 16. The plunger body 16 is formed in a rod shape. The plunger body 16 is inserted into the cylinder hole of the syringe body 11 through an opening formed at the other end portion of the syringe body 11 in the axial direction. Moreover, one end portion of the plunger body 16 in the axial direction contacts the gasket 13.

The operation portion 17 is formed at the other end portion of the plunger body 16 in the axial direction. The operation portion 17 is formed in a substantially discoid shape. When the medicine injection apparatus 1 is used, the operation portion 17 is pressed by a user. Accordingly, the one end portion of the plunger body 16 in the axial direction contacts the gasket 13 to slide the gasket 13 toward the discharge portion.

Moreover, various resins having been described as the material of the syringe body 11 can be applied as the material of the pusher member 4.

### [Syringe Holder]

Next, the syringe holder 5 will be described.

The syringe holder 5 is formed in a substantially cylindrical shape. The syringe holder 5 covers an outer peripheral surface of the syringe body 11 of the syringe 3 and an outer peripheral surface of the lockmechanism 12 of the syringe 3. Moreover, the syringe holder 5 is configured grippable by the user when the injection needle assembly 2 is attached to the syringe 3.

One end portion of the syringe holder 5 in the axial direction is provided with a viewing window 18. The viewing window 18 is provided at such a position that the liquid chamber 14 of the syringe 3 is visible from the outside of the syringe holder 5 when the syringe 3 is attached to the syringe holder 5. Thus, the inner visibility can be ensured even when the syringe holder 5 is attached to the syringe 3.

The other end of the syringe holder 5 in the axial direction is provided with a holder flange portion 19. The holder flange portion 19 protrudes substantially perpendicularly from part of an outer peripheral surface of the syringe holder 5. The holder flange portion 19 allows the user to grip the syringe holder 5, and can prevent fingers gripping the syringe holder 5 from slipping toward the other endportion in the axial direction upon administration of the medicine. The holder flange portion 19 can further prevent the medicine injection apparatus 1 from rolling over when the medicine injection apparatus 1 is placed on a desk, a table, etc.

Further, a middle portion of the syringe holder 5 in the axial direction is provided with the lock portion 5a. The lock portion 5a is an opening penetrating an outer wall of the syringe holder 5. The flange portion 15 of the syringe 3 is locked at the lock portion 5a.

By attachment of the syringe holder 5 to the syringe 3, the diameter of the medicine injection apparatus 1 can be increased, and the medicine injection apparatus 1 can be easily gripped. Thus, operability upon operation of the pusher member 4 is improved.

### [Injection Needle Assembly]

Next, the injection needle assembly 2 will be described.

As illustrated in Figs. 1 and 2, the injection needle assembly 2 includes a hollow needle tube 21 and a needle hub 22 configured to hold the needle tube 21.

### [Needle Tube]

As illustrated in Fig. 2, a needle tube with a size of 26 to 33 gauge (an outer diameter of 0.2 to 0.45 mm) according to ISO medical needle tube standards (IS09626:1991/Amd.1:2001(E)) is used as the needle tube 21, and preferably, a needle tube with a size of 30 to 33 gauge is used. Note that a needle thinner than 33 gauge may be used.

One end of the needle tube 21 is provided with a needle tip 21a having a blade surface. The other end of the needle tube 21 opposite to the needle tip 21a will be hereinafter referred to as a "base end." The axial length (hereinafter referred to as a "bevel length") of the needle tube 21 at the blade surface may be equal to or shorter than 1.4 mm (an adult) as the smallest thickness of the skin upper layer portion as described later, and may be equal to or longer than about 0.5 mm as the bevel length when a short bevel is formed at a 33-gauge needle tube. That is, the bevel length is preferably set within a range of 0.5 to 1.4 mm.

Further, the bevel length may be a smallest skin upper layer portion thickness of equal to or smaller than 0.9 mm (a child), i.e., may fall within a range of 0.5 to 0.9 mm. Note that the short bevel indicates a blade surface forming an angle of 18 to 25° with respect to a longitudinal direction of a needle generally used as an injection needle.

For example, the material of the needle tube 21 includes, but not limited to, stainless steel. Other metals such as aluminum, aluminum alloy, titanium, and titanium alloy can be used. Moreover, not only a straight needle but also a tapered needle formed at least partially in a tapered shape can be used as the needle tube 21. The tapered needle may have a greater diameter at a base end portion than at a needle tip end portion, and may have a tapered structure at a middle portion. Moreover, the sectional shape of the needle tube 21 may be not only a circular shape but also a polygonal shape such as a triangular shape.

### [Needle Hub]

The needle hub 22 includes a first member 23 configured to hold the needle tube 21, a second member 24 in which the discharge portion of the syringe 3 is fitted, the elastic member 25, a protector 26, a biasing member 27, and a restriction mechanism 28. The first member 23 and the second member 24 are formed as separate members. The materials of these first and second members 23, 24 include, for example, synthetic resins such as polycarbonate, polypropylene, and polyethylene.

The first member 23 includes a base portion 31, an adjustment portion 32, a stabilization portion 33, a guide portion 34, and a support portion 37. The base portion 31 is formed in a substantially circular columnar shape. The base portion 31 is provided with a housing recessed portion 36. The housing recessed portion 36 is formed in a substantially circular columnar shape recessed from one end to the other end of the base portion 31 in the axial direction. Moreover, the one end of the base portion 31 in the axial direction opens across an entire surface. The housing recessed portion 36 is provided with the support portion 37.

The support portion 37 has a first column portion 37A and a second column portion 37B. The first column portion 37A and the second column portion 37B are formed in a substantially circular columnar shape.

The first column portion 37A protrudes from a bottom surface 36a of the housing recessed portion 36 in the axial direction of the base portion 71. The second column portion 37B is provided at a center portion of one end surface 37aA of the first column portion 37A in the axial direction, and protrudes in the axial direction of the base portion 71. The center of the first column portion 37A and the center of the second column portion 37B are coincident with each other. The outer diameter of the second column portion 37B is set smaller than the outer diameter of the first column portion 37A.

Moreover, one end surface 37aB of the second column portion 37B in the axial direction is provided with the adjustment portion 32. The adjustment portion 32 is provided at a center portion of the one end surface 37aB of the second column portion 37B. Moreover, the center of the adjustment portion 32 is coincident with the centers of the first column portion 37A and the second column portion 37B.

A through-hole 39 through which the needle tube 21 penetrates is provided at the centers of the base portion 31, the support portion 37, and the adjustment portion 32. The base portion 31 is provided with an injection hole 44 for injection of an adhesive into the through-hole 39. The injection hole 44 opens at an outer peripheral surface of the base portion 31 on the other side with respect to the housing recessed portion 36 in the axial direction, and communicates with the through-hole 39. That is, the needle tube 21 is fixed to the base portion 31 and the support portion with the adhesive injected into the through-hole 39 through the injection hole 44.

The base end side of the needle tube 21 protrudes from an end surface 31a of the base portion 31 on the other side in the axial direction. The base portion 31 is, from the end surface 31a thereof, inserted into the second member 24, and the base end side of the needle tube 21 is inserted into an insertion hole of the elastic member 25. Then, the end surface 31a of the base portion 31 contacts the end surface of the elastic member 25.

Moreover, the outer peripheral surface of the base portion 31 is provided with a connection piece 35. The connection piece 35 is, on one end side of the base portion 31 in the axial direction, formed as a ring-shaped flange portion protruding outward in a radial direction of the base portion 31. The connection piece 35 has flat surfaces 35a, 35b facing the axial direction of the base portion 31. The second member 24 is connected to the flat surface 35b of the connection piece 35. Moreover, a tip end portion of the connection piece 35 serves as the guide portion 34. The guide portion 34 will be described in detail later.

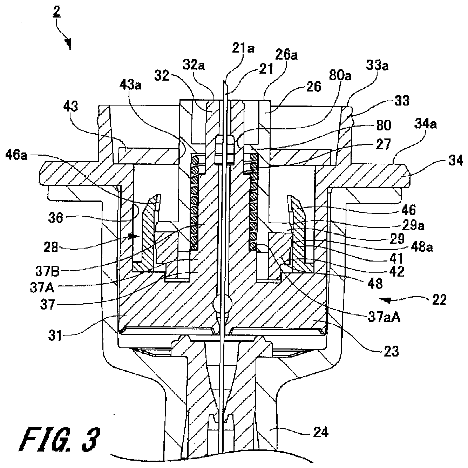

An end surface of the adjustment portion 32 forms a needle protruding surface 32a from which the needle tip 21a of the needle tube 21 protrudes. The needle protruding surface 32a is formed as a flat surface perpendicular to the axial direction of the needle tube 21. When the skin upper layer portion is punctured with the needle tube 21, the needle protruding surface 32a contacts the skin surface to define the depth of puncturing with the needle tube 21. That is, the depth of puncturing of the skin upper layer portion with the needle tube 21 is determined by the length (hereinafter referred to as a "protruding length L") of the needle tube 21 protruding from the needle protruding surface 32a (see Fig. 3).

The thickness of the skin upper layer portion is equivalent to a depth from the skin surface to a dermis layer, and falls substantially within a range of 0.5 to 3.0 mm. Thus, the protruding length L of the needle tube 21 can be set within a range of 0.5 to 3.0 mm.

A region to which an influenza vaccine is administered is generally the deltoid muscle. For this reason, the thickness of a skin upper layer portion of the deltoid muscle has been measured for 19 children and 31 adults. Such measurement has been performed in such a manner that contrast radiography of the skin upper layer portion with a high ultrasonic reflectivity is performed using an ultrasonic measurement apparatus (NP60R-UBM high-resolution echo for small animals, Nepa Gene Co., Ltd.). Note that a measurement value was in log-normal distribution, and therefore, a range of MEAN±2SD was obtained by geometric averaging.

As a result, the thickness of the skin upper layer portion at the deltoid muscle of the child was 0.9 to 1.6 mm. Moreover, the thickness of the skin upper layer portion at the deltoid muscle of the adult was 1.4 to 2.6 mm at a distal portion, 1.4 to 2.5 mm at a center portion, and 1.5 to 2.5 mm at a proximal portion. From the above-described points, it has confirmed that the thickness of the skin upper layer portion at the deltoid muscle is equal to or greater than 0.9 mm in the case of the child, and is equal to or greater than 1.4 mm in the case of the adult. Thus, upon injection into the skin upper layer portion of the deltoid muscle, the protruding length L of the needle tube 21 is preferably set within a range of 0.9 to 1.4 mm.

With such a setting of the protruding length L, the blade surface of the needle tip 21a can be positioned at the skin upper layer portion. As a result, even when a needle hole (a medicine discharge port) opening at the blade surface is at any position within the blade surface, the needle hole is positioned at the skin upper layer portion. Note that even when the medicine discharge port is positioned at the skin upper layer portion, if the needle tip 21a pierces deeper than the skin upper layer portion, the medicine flows into a subcutaneous tissue through a portion between a side surface of an end portion of the needle tip 21a and the cut skin, and therefore, it is important that the blade surface is reliably positioned at the skin upper layer portion.

Note that in the case of use for administration to the skin upper layer portion, it is difficult for a needle tube with a size greater than 26 gauge to have a bevel length of equal to or smaller than 1.0 mm. For this reason, a needle tube with a size smaller than 26 gauge is preferably used for setting the protruding length L of the needle tube 21 within a preferable range (0.9 to 1.4 mm).

The needle protruding surface 32a of the adjustment portion 32 is formed such that a distance S from a peripheral edge of the needle protruding surface 32a to an outer peripheral surface of the needle tube 21 is equal to or shorter than 1.4 mm, and preferably within a range of 0.3 to 1.4 mm. The distance S from the peripheral edge of the needle protruding surface 32a to the peripheral surface of the needle tube 21 is set considering pressure application to a blister formed by medicine administration to the skin upper layer portion. That is, the needle protruding surface 32a is set to such a size that the needle protruding surface 32a is sufficiently smaller than the blister formed at the skin upper layer portion, and therefore, does not interfere with formation of the blister. This can prevents, as a result, the administered medicine from leaking even when the needle protruding surface 32a presses the skin around the needle tube 21.

The stabilization portion 33 is formed in a tubular shape protruding from the flat surface 35a of the connection piece 35. The needle tube 21, the adjustment portion 32, and the later-described protector 26 are arranged in a cylinder hole of the stabilization portion 33. That is, the stabilization portion 33 is formed in the tubular shape covering the periphery of the adjustment portion 32 through which the needle tube 21 penetrates and the periphery of the protector 26. Moreover, the stabilization portion 33 is provided apart from the needle tip 21a of the needle tube 21 in the radial direction.

An end surface 33a of the stabilization portion 33 is positioned on the base end side of the needle tube 21 with respect to the needle protruding surface 32a of the adjustment portion 32. When a biological body is punctured with the needle tip 21a of the needle tube 21, the needle protruding surface 32a first contacts the surface skin, and then, the end surface 33a of the stabilization portion 33 contacts the skin surface. In this state, the end surface 33a of the stabilization portion 33 contacts the skin so that the medicine injection apparatus 1 can be stabilized and that the needle tube 21 can be held in a posture substantially perpendicular to the skin.

Note that even when positioned on the same plane as that of the needle protruding surface 32a or positioned closer to the needle tip 21a of the needle tube 21 with respect to the needle protruding surface 32a, the end surface 33a of the stabilization portion 33 can hold the needle tube 21 in the posture substantially perpendicular to the skin. Considering the bulging skin when the stabilization portion 33 is pressed against the skin, an axial distance between the end surface 33a of the stabilization portion 33 and the needle protruding surface 32a is preferably set to equal to or smaller than 1.3 mm.

Moreover, the inner diameter d of the stabilizationportion 33 is set to a value equal to or greater than the diameter of the blister formed at the skin. Specifically, a distance T from an inner wall surface of the stabilization portion 33 to the peripheral edge of the needle protruding surface 32a is set within a range of 4 mm to 15 mm. This can prevent interference with formation of the blister without pressure application to the blister from the inner wall surface of the stabilization portion 33.

The distance T from the inner wall surface of the stabilization portion 33 to the needle protruding surface 32a has no upper limit as long as such a distance is equal to or longer than 4 mm. However, a longer distance T results in a greater outer diameter of the stabilization portion 33. Thus, in the case of puncturing, e.g., a thin arm of a child with the needle tube 21, it is difficult that the entirety of the end surface 33a of the stabilization portion 33 contacts the skin. For this reason, it is preferably defined that 15 mm is the maximum distance T, considering the thickness of the arm of the child.

As long as the distance S from the needle protruding surface 32a to the outer peripheral surface of the needle tube 21 is equal to or longer than 0.3 mm, the adjustment portion 32 does not penetrate the skin. Thus, the inner diameter d of the stabilization portion 33 can be set to equal to or greater than 9 mm, considering the distance T (equal to or longer than 4 mm) from the inner wall surface of the stabilization portion 33 to the peripheral edge of the needle protruding surface 32a and the diameter (about 0.3 mm) of the needle protruding surface 32a.

Note that the shape of the stabilization portion 33 is not limited to the cylindrical shape, and the stabilization portion 33 may be formed in a rectangular cylindrical shape such as a quadrangular or hexagonal column having a cylinder hole at the center, for example.

The guide portion 34 is a tip-end-side portion of the connection piece 35 positioned on the outside of the first member 23 in the radial direction with respect to the stabilization portion 33. The guide portion 34 has a contact surface 34a configured to contact the skin. The contact surface 34a is part of the flat surface 35a of the connection piece 35, and is a flat surface substantially parallel to the end surface 33a of the stabilization portion 33. The stabilization portion 33 is pressed until the contact surface 34a of the guide portion 34 contacts the skin, and therefore, a force of equal to or greater than a predetermined value can be constantly ensured as the force of pressing the skin by the stabilization portion 33 and the needle tube 21. Thus, the skin is reliably punctured with a portion (equivalent to the protruding length L) protruding from the needle protruding surface 32a of the needle tube 21.

A distance (hereinafter referred to as a "guide portion height") Y from the contact surface 34a of the guide portion 34 to the end surface 33a of the stabilization portion 33 is set so that the skin can be, with proper pressing force, pressed and punctured with the needle tube 21 and the stabilization portion 33. Note that the proper pressing force of the needle tube 21 and the stabilization portion 33 is 3 to 20 N, for example. Thus, the pressing force of the needle tube 21 and the stabilizationportion 33 on the skin is guided by the guide portion 34 so that the needle tip 21a of the needle tube 21 can be reliably positioned at the skin upper layer portion and that a feeling of security can be provided to the user.

The guide portion height Y is, as necessary, determined based on the inner diameter d of the stabilization portion 33 and a length (hereinafter referred to as a "guide portion length") X from a tip end surface of the guide portion 34 to an outer peripheral surface of the stabilization portion 33. For example, when the inner diameter d of the stabilization portion 33 is 12 mm and the guide portion length X is 3.0 mm, the guide portion height Y is set within a range of 2.3 to 6.6 mm.

Next, the protector 26 will be described. As illustrated in Figs. 1 and 2, the protector 26 covers the periphery of the second column portion 37B, the periphery of the adjustment portion 32 through which the needle tube 21 penetrates, and the periphery of the needle tip 21a of the needle tube 21 in a state before the skin is punctured with the needle tube 21. Moreover, the protector 26 is formed in a cylindrical shape.

The protector 26 is supported by the support portion 37 and a retaining member 43 to move in the axial directions of these components (the axial direction of the needle tube 21). Part of the protector 26 on the other side in the axial direction is inserted into a space 40 formed between the housing recessed portion 36 and the support portion 37.

The thickness of the protector 26 is preferably set sufficiently smaller than the diameter of the needle protruding surface 32a for preventing interference with formation of the blister.

The protector 26 is provided with a contact piece 29 and a spring contact portion 30. The contact piece 29 is disposed opposite to the needle tip 21a of the needle tube 21 at the protector 26, i.e., disposed on the other end side in the axial direction at the protector 26. The contact piece 29 is formed as a ring-shaped flange portion protruding outward in the radial direction from an outer peripheral surface of the protector 26.

Moreover, an outer edge of the contact piece 29 in the radial direction is provided with an inclined surface 29a. The inclined surface 29a is provided at a corner portion of the outer edge of the contact piece 29 close to the needle tip 21a of the needle tube 21. The inclined surface 29a is formed in such a tapered shape that the diameter thereof is continuously decreased from the other side to the one side in the axial direction.

The spring contact portion 30 is a ring-shaped inner flange provided continuously to an inner wall of the protector 26 in the circumferential direction and protruding inward in the radial direction of the protector 26. An insertion hole 30a into which the adjustment portion 32 and the needle tip 21a of the needle tube 21 are inserted is provided on the inside of the spring contact portion 30 in the radial direction.

The shape of the protector 26 is not limited to the cylindrical shape, and may be formed in a rectangular cylindrical shape such as a quadrangular or hexagonal column having a cylinder hole at the center, for example.

Moreover, a lock member 41 and an expansion member 42 of the restriction mechanism 28 as described later are arranged on the other side in the axial direction at the protector 26.

In the cylinder hole of the protector 26, the biasing member 27 is disposed to cover the periphery of the second column portion 37B of the support portion 37 and the periphery of the adjustment portion 32. One end portion of the biasing member 27 in the axial direction contacts the spring contact portion 30, and the other end portion of the biasing member 27 in the axial direction contacts the one end surface 37aA of the first column portion 37A.

The biasing member 27 is a compression coil spring, and biases the protector 2 6 toward the one side in the axial direction, i.e. , toward the needle tip 21a of the needle tube 21. Biasing force of the biasing member 27 is set smaller than the proper pressing force when the skin is punctured with the needle tube 21, and is set to equal to or smaller than 3 N, for example. This eliminates a cause for interference of the biasing force of the biasing member 27 with positioning of the needle tip 21a of the needle tube 21 at the skin upper layer portion when the skin is punctured with the needle tube 21, and therefore, the needle tip 21a of the needle tube 21 can be reliably positioned at the skin upper layer portion.

Note that in the present embodiment, the example where the compression coil spring is applied as the biasing member 27 has been described, but the present invention is not limited to such an example. As long as the biasing member is an elastic member configured to elastically deform upon application of predetermined pressing force, various other spring members such as a plate spring, sponge, gel, and a rubber member can be applied as the biasing member, for example.

Next, the restriction mechanism 28 will be described. The restriction mechanism 28 has the lock member 41 and the expansion member 42. The lockmember 41 has a cylindrical portion 45 formed in a substantially cylindrical shape, and a plurality of lock portions 46.

The lock member 41 is disposed in the housing recessed portion 36. Moreover, the cylindrical portion 45 is fixed to the bottom surface 36a of the housing recessed portion 36. Further, the lock member 41 is disposed to cover the periphery of the other side of the support portion 37 in the axial direction. The plurality of lock portions 46 are formed continuously on one side of the cylindrical portion 45 in the axial direction.

The plurality of lock portions 46 are provided at predetermined intervals in the circumferential direction on one end surface of the cylindrical portion 45 in the axial direction. The plurality of lock portions 46 protrude in a substantially flat plate shape from the one end surface of the cylindrical portion 45 in the axial direction. Moreover, the plurality of lock portions 46 are formed in a tongue shape, and exhibit elasticity.

A tip end portion of each lock portion 46 is inclined with respect to the axial direction of the needle tube 21 to approach inward in the radial direction with respect to a base end portion of the lock portions 46 close to the cylindrical portion 45, i.e., approach the support portion 37. A lock piece 46a is provided on an inner surface of the tip end portion of each lock portion 46 in the radial direction. The lock piece 4 6a protrudes substantially perpendicularly from the inner surface of the lock portion 46 to the inside in the radial direction.

Moreover, the diameter of a space formed by the plurality of lock portions 46 in a natural state in which the plurality of lock portions 46 are not elastically deformed is set slightly smaller than the outer diameter of the contact piece 29 of the protector 26. Further, the expansion member 42 is disposed on the inside of the plurality of lock portions 46 of the lock member 41.

The expansion member 42 is formed in a substantially tubular shape. The expansion member 42 is detachably attached to an outer peripheral portion of the protector 26 on the other side in the axial direction with respect to the contact piece 29. An expansion portion 48 is provided on one side of the expansion member 42 in the axial direction.

The expansion portion 48 protrudes outward in the radial direction from an outer peripheral surface of the expansion member 42. One end portion of the expansion portion 48 in the axial direction has an outer diameter continuously increased from the other side to the one side of the expansion member 42 in the axial direction. An expansion surface 48a of the expansion portion 48 inclined with respect to the axial direction is held by the plurality of lock portions 46 of the lock member 41.

Of the expansion portion 48, a portion with the greatest outer diameter is formed such that the diameter (hereinafter referred to as a "maximum outer diameter") of such a portion is set greater than the diameter of the space formed by the plurality of lock portions 46 in the natural state in which the plurality of lock portions 46 are not elastically deformed. Further, the maximum outer diameter of the expansion portion 48 is set greater than the outer diameter of the contact piece 29 of the protector 26. In addition, the one end portion of the expansion portion 48 of the expansion member 42 in the axial direction is, after puncturing, locked at the lock piece 46a provided at each lock portion 46 of the lock member 41.

The first member 23 has the retaining member 43. The retaining member 43 is formed in a substantially discoid shape. The retaining member 43 is fixed to the base portion 31 to close an opening of the housing recessed portion 36 on the one side in the axial direction. A support hole 43a opens at a center portion of the retaining member 43. The adjustment portion 32, the support portion 37, and the protector 26 are inserted into the support hole 43a. Moreover, the retainingmember 43 supports the protector 26 such that the protector 26 moves along the axial direction of the support portion 37, i.e., the axial direction of the needle tube 21.

In the state before the skin is punctured with the needle tube 21, the contact piece 29 of the protector 26 contacts the other end surface of the retaining member 43 in the axial direction. This prevents the protector 26 biased by the biasing member 27 from dropping out of the first member 23.

### [Second Member]

Next, the second member 24 will be described. The second member 24 is formed in a tubular shape. One end portion of the second member 24 in the axial direction serves as an insertion portion 51 into which the base portion 31 of the first member 23 is inserted, and the other end portion of the second member 24 in the axial direction serves as a fitting portion 52 in which the discharge portion of the syringe 3 is fitted. A cylinder hole 51a of the insertion portion 51 is set to a size corresponding to the base portion 31 of the first member 23.

An outer peripheral surface of one end portion of the insertion portion 51 in the axial direction of the second member 24 is provided with a fixing piece 54. The fixing piece 54 is formed as a ring-shaped flange formed continuously to a tip end of the insertion portion 51 and protruding outward in the radial direction. The flat surface 35b of the connection piece 35 provided at the first member 23 is fixed in contact with the fixing piece 54. The method for fixing the fixing piece 54 and the connection piece 35 together includes, for example, an adhesive, ultrasonic welding, laser welding, and a fixing screw.

The outer diameter of the fitting portion 52 is set smaller than the outer diameter of the insertion portion 51. Further, a cylinder hole 52a of the fitting portion 52 is set to a size corresponding to the discharge portion of the syringe 3, and has a diameter continuously decreased toward the insertion portion 51. In addition, an outer peripheral surface of the fitting portion 52 is provided with an external thread portion 52b for threadedly-engagement with the lock mechanism 12 of the syringe 3 (see Fig. 1). Moreover, the elastic member 25 is disposed between the cylinder hole 51a of the insertion portion 51 and the cylinder hole 52a of the fitting portion 52.

### [Elastic Member]

Next, the elasticmember 25 will be described. The elastic member 25 is formed of an elastically-deformable member. The material of the elasticmember 25 is an elasticmaterial including, for example, various rubber materials such as natural rubber, silicone rubber, and isobutylene rubber, various thermoplastic elastomers such as polyurethane-based elastomer and styrene-based elastomer, and a mixture thereof.

The elastic member 25 is disposed in the second member 24, and is interposed between the first member 23 and the syringe 3. Thus, a clearance formed between a base-end-side outer peripheral surface of the needle tube 21 protruding from the first member 23 and the second member 24 is filled. When the discharge portion of the syringe 3 is fitted in the second member 24, the elastic member 25 elastically deforms to closely contact the outer peripheral surface of the needle tube 21 in a liquid-tight manner. Thus, the medicine filling the syringe 3 penetrates into between the needle tube 21 and the elastic member 25, and therefore, leakage toward the first member 23 can be prevented.

### 1-2. Method for Using Medicine Injection Apparatus

Next, the method for using the medicine injection apparatus 1 having the above-described configuration will be described with reference to Figs. 1 to 4.

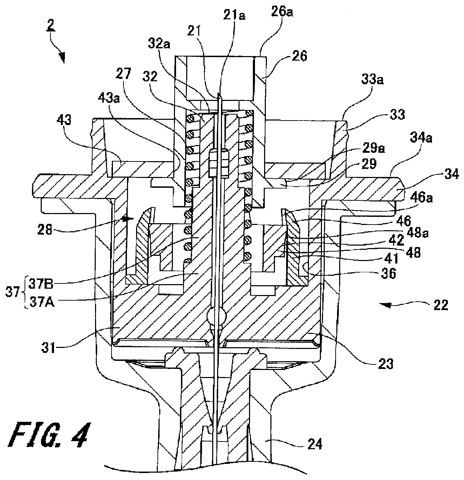

Fig. 3 is a sectional view of a main portion of the medicine injection apparatus 1 during puncturing, and Fig. 4 is a sectional view of the main portion of the medicine injection apparatus 1 after puncturing.

First, as illustrated in Figs. 1 and 2, the syringe 3 is attached to the injection needle assembly 2 in advance. Specifically, the discharge portion of the syringe 3 is inserted into the fitting portion 52 of the second member 24, and the lock mechanism 12 is threadedly engaged with the external thread portion 52b. Thus, attachment of the injection needle assembly 2 to the syringe 3 is completed.

In this state, the protector 26 is biased toward the one end side in the axial direction by the biasing member 27, and the contact piece 29 contacts the retaining member 43. This prevents the protector 26 from dropping out of the support portion 37 of the first member 23. The position of the protector 26 in this state is a first position.

Further, the needle tip 21a of the needle tube 21 is covered with the protector 26, and is housed in the cylinder hole of the protector 26. This can prevent, in the state before puncturing, puncturing with the needle tip 21a of the needle tube 21 despite user's intention.

Next, the end surface 33a of the stabilization portion 33 is positioned facing the skin. Thus, the needle tip 21a of the needle tube 21 is positioned facing the skin to be punctured. Next, the medicine injection apparatus 1 is moved substantially perpendicular to the skin. Thus, a tip end surface 26a of the protector 26 is pressed against the skin. When the medicine injection apparatus 1 is further pressed onto the skin against the biasing force of the biasing member 27, the protector 26 moves along the axial direction of the support portion 37. Thus, the needle protruding surface 32a of the adjustment portion 32 and the needle tip 21a of the needle tube 21 protrude from one end of the protector 26 in the axial direction, i.e., from the tip end surface 26a, and therefore, the needle tip 21a of the needle tube 21 is exposed. The position of the protector 26 in this state is a second position.

The outer peripheral surface of the protector 26 is supported by the support hole 43a of the retaining member 43. Thus, the protector 26 can be smoothly moved without rattling.

When the protector 26 moves from the one side to the other side in the axial direction of the support portion 37, the expansion member 42 is pressed against the contact piece 29 of the protector 26, and moves, together with the protector 26, from the one side to the other side in the axial direction of the support portion 37. When the expansion member 42 moves from the one side to the other side in the axial direction of the support portion 37, the plurality of lock portions 46 contacting the expansion surface 48a of the expansion portion 48 are pressed against the expansion portion 48.

The one end portion of the expansion portion 48 in the axial direction is formed such that the outer diameter thereof is continuously increased from the other side to the one side in the axial direction of the expansion member 42. Thus, the plurality of lock portions 46 are pressed outward in the radial direction. Then, the plurality of lock portions 46 elastically deform to rotate about a pivot point, i.e., base end portions close to the cylindrical portion 45, and the opening surrounded by the plurality of lock portions 46 is expanded outward in the radial direction. Thus, as illustrated in Fig. 3, the contact piece 29 of the protector 26 is, together with expansion member 42, inserted into the lock member 41 without being caught by the plurality of lock portions 46.

When the expansion portion 48 of the expansion member 42 and the contact piece 29 of the protector 26 move over the lock pieces 46a of the plurality of lock portions 46, the pressing force on the plurality of lock portions 46 is released. Then, the plurality of lock portions 46 restore to an original shape (the natural state) by elasticity of the plurality of lock portions 46 themselves. In this state, the biasing member 27 is elastically deformed and compressed between the springcontact portion 30 of the protector 26 and one end surface 77aA of the first column portion 37A.

The medicine injection apparatus 1 is further moved substantially perpendicular to the skin. Thus, the skin is punctured with the needle tip 21a, and the end surface 33a of the stabilization portion 33 is pressed against the skin. In this state, the needle protruding surface 32a can contact the skin to flatly deform the skin, and therefore, the skin can be punctured with the needle tip 21a of the needle tube 21 by the protruding length L.

Next, the end surface 33a of the stabilization portion 33 is pressed until the contact surface 34a of the guide portion 34 contacts the skin. In this state, the guide portion height Y (see Fig. 2) is set so that the skin can be, with the proper pressing force, punctured with the needle tube 21 and the stabilization portion 33. Thus, the force of pressing the skin by the stabilization portion 33 reaches the predetermined value.

As a result, the user can recognize the proper pressing force of the stabilization portion 33, and the needle tip 21a and the blade surface of the needle tube 21 can be reliably positioned at the skin upper layer portion. Since the guide portion 34 serves as a mark for recognition of the proper pressing force of the stabilization portion 33 as described above, the user can use the medicine injection apparatus 1 with security.

Moreover, the stabilization portion 33 contacts the skin so that the posture of the medicine injection apparatus 1 can be stabilized and that the skin can be punctured straight with the needle tube 21. Further, shaking caused at the needle tube 21 after puncturing can be prevented, and therefore, stable medicine administration can be performed.

Further, in the case of, e.g., a needle tube with a very short protruding length of about 0.5 mm, the skin is not punctured with a needle tip even when the needle tip contacts the skin. However, the skin pressed against the stabilization portion 33 is pressed down perpendicularly, leading to a state in which the skin on the inside of the stabilization portion 33 is pulled such that tension is applied to the skin. Thus, the skin is less slipped from the needle tip 21a of the needle tube 21. With the stabilization portion 33, the effect of more easily puncturing the skin with the needle tip 21a can be obtained.

In addition, in a case where the above-described configuration is applied to a hypodermic injection apparatus configured to puncture a layer below the skin upper layer portion with a needle tube, the length of the needle tube piercing the skin is longer than that of the injection needle assembly 2 configured to puncture the skin upper layer portion with the needle tube 21 according to the present embodiment, and therefore, the length of a protector 26 in the axial direction is increased. Thus, not only the axial length of a support portion 37 configured to support the protector 26 but also the moving distance of the protector 26 along the axial direction of the support portion 37 are increased. As a result, in the hypodermic injection apparatus, there are defects that the axial length of a housing recessed portion 36 provided at a base portion 31 needs to be long and that a first member and an injection needle assembly are enlarged.

On the other hand, the injection needle assembly 2 of the present embodiment is configured such that the skin upper layer portion is punctured with the needle tip 21a of the needle tube 21, and therefore, the protruding length L of the needle tube 21 is an extremely short length of 0.5 to 3.0 mm. Thus, the length of the protector 26 in the axial direction can be also shortened, and the moving distance of the protector 26 along the axial direction of the support portion 37 canbe also extremely shortened. As a result, even when the above-described configuration is applied to the injection needle assembly 2, enlargement of the entirety of the injection needle assembly 2 can be prevented.

Further, the injection needle assembly 2 of the present embodiment is configured such that the biasing member 27 is housed in the cylinder hole of the protector 26 and is disposed close to the second column portion 77B, i.e., one side of the support portion 37 in the axial direction. Thus, the length of the housing recessed portion 36 in the axial direction can be more shortened. Moreover, the axial length of the lock member 41 housed in the housing recessed portion 36 can be also shortened, leading to size reduction of the injection needle assembly 2.

After the skin has been punctured with the needle tip 21a of the needle tube 21, the pusher member 4 (see Fig. 1) is pressed to move the gasket 13 toward the discharge portion. Thus, the medicine filling the liquid chamber 14 of the syringe 3 is pushed out of the discharge portion, and then, is injected from the needle tip 21a to the skin upper layer portion through the needle hole of the needle tube 21. In this state, no space is formed between a tip end of the discharge portion and the base end of the needle tube 21, and therefore, a remaining medicine amount can be reduced.

Upon completion of medicine administration, the medicine injection apparatus 1 is separated from the skin, and then, the end surface 33a of the stabilization portion 33, the needle protruding surface 32a, and the tip end surface 26a of the protector 26 are separated from the skin. In this state, the biasing member 27 is released from pressing from the skin through the protector 26. Then, the protector 26 is biased toward the needle tip 21a of the needle tube 21 by restorative force (the biasing force) of the biasing member 27. Thus, as illustrated in Fig. 4, the protector 26 moves from the other side to the one side in the axial direction of the support portion 37 with the protector 26 being supported by the support hole 43a of the retaining member 43.

When the protector 26 moves from the other side to the one side in the axial direction of the support portion 37, the inclined surface 29a of the contact piece 29 contacts the lock pieces 46a of the plurality of lock portions 46. The inclined surface 29a is formed in such a tapered shape that the diameter thereof is continuously decreased from the other side to the one side in the axial direction. Thus, the plurality of lock portions 46 are pressed outward in the radial direction, and therefore, the space surrounded by the plurality of lock portions 4 6 is expanded outward in the radial direction. Thus, the contact piece 29 can pass through the inside of the plurality of lock portions 4 6 without being caught by the plurality of lock portions 46. Moreover, after the contact piece 29 has been passed, the pressing force on the plurality of lock portions 46 is released, and then, the plurality of lock portions 46 restore to the original shape (the natural state) by the elasticity of the plurality of lock portions 46 themselves.

Then, the contact piece 29 contacts the retaining member 43, and therefore, movement of the protector 26 in the axial direction is restricted. This also prevents the protector 26 from dropping out of the support portion 37 of the first member 23.

The protector 26 covers the periphery of the needle tip 21a of the needle tube 21, and the needle tube 21 is housed in the protector 26. That is, the protector 26 returns from the second position to the first position. Thus, the protector 26 can be automatically moved in response to puncturing operation, and the periphery of the needle tip 21a of the needle tube 21 can be easily covered.

On the other hand, the expansion member 42 is configured such that the maximum outer diameter of the expansion portion 48 is set greater than the outer diameter of the contact piece 29 of the protector 26. Moreover, the one end portion of the expansion portion 48 in the axial direction is locked at the lock pieces 46a of the plurality of lock portions 46. Thus, the expansion member 42 is detached from the protector 26, and remains in the lock member 41.

The expansion member 42 remains in the lock member 41. Thus, the plurality of lock portions 46 are pressed outward in the radial direction, and therefore, the space surrounded by the plurality of lock portions 46 is not expanded outward in the radial direction. In the case of pressing the protector 26 from the one side to the other side in the axial direction of the support portion 37, the contact piece 29 contacts the plurality of lock portions 46 of the lock member 41. Thus, movement of the protector 26 from the one side to the other side in the axial direction after puncturing is restricted. This can prevent, after puncturing, the needle tip 21a of the needle tube 21 from protruding from the tip end surface 26a of the protector 26 again. As a result, the needle tip 21a of the needle tube 21 after use can be held in a safe state, and therefore, puncturing with the needle tip 21a of the needle tube 21 after use despite the user's intention can be prevented.

Further, the needle tip 21a of the needle tube 21 after use is covered with the protector 26. Thus, scattering of blood adhering to the needle tip 21a can be prevented, and inflection via the blood can be prevented.

In addition, the elastically-deformable biasing member 27 is housed in the cylinder hole of the protector 26, and is disposed on a tip end side of the first member 23 in the axial direction. Thus, the biasing member 27 can be separated from the lock member 41 and the expansion member 42 of the restriction mechanism 28. With this configuration, contact among the members can be prevented when the biasing member 27 elastically deforms, when the expansion member 42 moves, when the protector 26 moves, or when the plurality of lock portions 46 of the lock member 41 elastically deform. As a result, movement operation of the protector 26 can be smoothly performed.

### 2. Second Embodiment

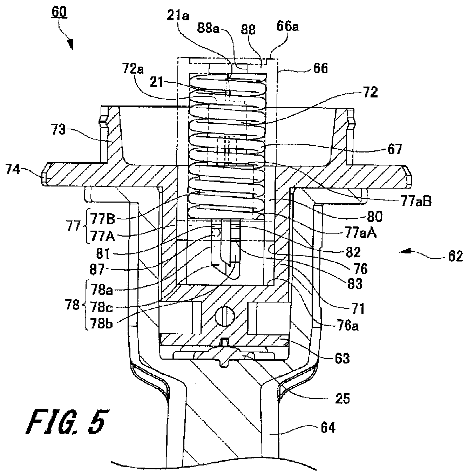

Next, a medicine injection apparatus of a second embodiment will be described with reference to Figs. 5 to 7.

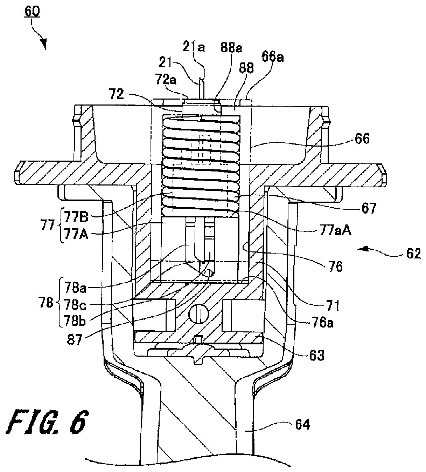

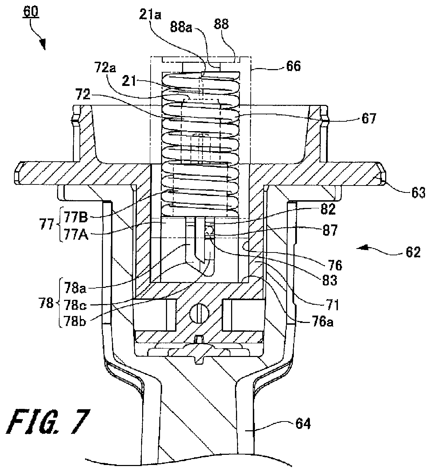

Fig. 5 is a sectional view in a state before puncturing, Fig. 6 is a sectional view in a state during puncturing, and Fig. 7 is a sectional view in a state after puncturing.

The medicine injection apparatus of the second embodiment is different from the medicine injection apparatus 1 of the first embodiment in a configuration of a first member in an injection needle assembly. Thus, the first member will be mainly described herein. The same reference numerals are used to represent common elements to those of the injection needle assembly 2 of the first embodiment, and overlapping description thereof will not be repeated.

As illustrated in Fig. 5, an injection needle assembly 60 includes a hollow needle tube 21 and a needle hub 62 configured to hold the needle tube 21. The needle hub 62 includes a first member 63, a second member 64 in which a discharge portion of a syringe 3 is fitted, an elastic member 25, a protector 66, and a biasing member 67.

The first member 63 includes a base portion 71, an adjustment portion 72, a stabilization portion 73, a guide portion 74, and a support portion 77. The base portion 71 is formed in a substantially circular columnar shape. The base portion 71 is provided with a housing recessed portion 76. One end of the base portion 71 in an axial direction opens across an entire surface. The housing recessed portion 76 is formed in a substantially circular columnar shape recessed from the one end to the other end in the axial direction of the base portion 71. The housing recessedportion 76 is provided with the support portion 77. The support portion 77 protrudes from a center portion of a bottom surface 76a of the housing recessed portion 76 in the axial direction of the base portion 71.

The support portion 77 has a first column portion 77A and a second column portion 77B. The first column portion 77A and the second column portion 77B are formed in a substantially circular columnar shape. A side surface of the first column portion 77A is provided with a slide groove 78.

The slide groove 78 has a first slide portion 78a, a second slide portion 78b, and an inclined portion 78c allowing communication between the first slide portion 78a and the second slide portion 78b. The first slide portion 78a, the second slide portion 78b, and the inclined portion 78c are groove portions recessed inward in a radial direction from a side surface of the support portion 77. Moreover, the length, i.e., the groove length, of each of the first slide portion 78a and the inclined portion 78c from the side surface of the support portion 77 toward the inside in the radial direction is set smaller than the groove depth of the second slide portion 78b. Moreover, a slide protrusion 87 of the protector 66 as described later is slidably inserted into the first slide portion 78a, the second slide portion 78b, and the inclined portion 78c.

The first slide portion 78a and the second slide portion 78b are, in parallel with the axial direction of the first column portion 77A, formed from an outer edge of one end surface 77aA of the first column portion 77A. Moreover, the first slide portion 78a and the second slide portion 78b are provided with a predetermined spacing in a circumferential direction of the support portion 77.

Moreover, the other end portions of the first slide portion 78a and the second slide portion 78b in the axial direction of the support portion 77 are provided continuously to the inclined portion 78c. The slide groove 78 is set such that the length of extension of the second slide portion 78b along the axial direction of the support portion 77 is longer than the length of extension of the first slide portion 78a along the axial direction of the support portion 77. Thus, an end portion of the inclined portion 78c close to the first slide portion 78a is positioned on one side of the support portion 77 in the axial direction with respect to an end portion of the inclined portion 78c close to the second slide portion 78b. That is, the inclined portion 78c is inclined from the one side to the other side in the axial direction of the support portion 77 while extending from the first slide portion 78a to the second slide portion 78b along the circumferential direction of the support portion 77. Thus, the inclined portion 78c is inclined with respect to the axial direction and the circumferential direction of the support portion 77.

One end portion of the first slide portion 78a in the axial direction of the first column portion 77A is provided with a first stopper 81. The first stopper 81 is a protrusion extending outward in the radial direction of the first column portion 77A from a bottom surface of the first slide portion 78a. The slide protrusion 87 of the protector 66 as described later contacts the first stopper 81.

One end portion of the second slide portion 78b in the axial direction of the first column portion 77A is provided with a second stopper 82 and a return restriction portion 83. The second stopper 82 and the return restriction portion 83 are protrusions extending from a bottom surface of the second slide portion 78b in the radial direction of the first column portion 77A.

The return restriction portion 83 is provided on the other side in the axial direction of the first column portion 77A with respect to the second stopper 82. Moreover, an end surface of the return restriction portion 83 on the other side in the axial direction of the first column portion 77A forms an inclined surface, and an end surface of the return restriction portion 83 on the one side in the axial direction stands substantially perpendicularly from the bottom surface of the second slide portion 78b. The slide protrusion 87 of the protector 66 as described later contacts the second stopper 82 and the return restriction portion 83.

One end surface 77aB of the second column portion 77B in the axial direction is provided with the adjustment portion 72. The adjustment portion 72 is provided at a center portion of the one end surface 77aB of the second column portion 77B. Moreover, the center of the adjustment portion 72 is coincident with the centers of the first column portion 77A and the second column portion 77B.

Next, the protector 66 will be described. The protector 66 is formed in a tubular shape. In the state before the skin is punctured with the needle tube 21, the protector 66 covers the periphery of the second column portion 77B, the periphery of the adjustment portion 72 through which the needle tube 21 penetrates, and the periphery of a needle tip 21a of the needle tube 21. The protector 66 is supported by the support portion 77 to move in the axial direction of the support portion 77 (the axial direction of the needle tube 21) and to rotate in the circumferential direction of the support portion 77 (the circumferential direction of the needle tube 21). Part of the protector 66 on the other side in the axial direction is inserted into a space 80 formed between the housing recessed portion 76 and the support portion 77.

An inner wall of a cylinder hole of the protector 66 is provided with the slide protrusion 87 and a spring contact portion 88. The spring contact portion 88 is disposed at one end portion of the protector 66 in the axial direction, and the slide protrusion 87 is disposed at the other end portion of the protector 66 in the axial direction.

The slide protrusion 87 protrudes inward in the radial direction of the protector 66 from the inner wall of the protector 66. Moreover, the slide protrusion 87 is slidably inserted into the slide groove 78.

In the state before puncturing, the slide protrusion 87 is positioned at the first slide portion 78a of the slide groove 78. When the skin is punctured with the needle tube 21, the slide protrusion 87 is positioned at the inclined portion 78c of the slide groove 78 (see Fig. 6). After puncturing, the slide protrusion 87 is positioned between the second stopper 82 and the return restriction portion 83 at the second slide portion 78b of the slide groove 78 (see Fig. 7). Thus, the slide protrusion 87 and the return restriction portion 83 form a restriction mechanism.

The spring contact portion 88 is an inner flange provided continuously in the circumferential direction of the inner wall of the protector 66 and protruding inward in the radial direction of the protector 66. The inside of the spring contact portion 88 in the radial direction is provided with an insertion hole 88a into which the adjustment portion 72 and the needle tip 21a of the needle tube 21 are inserted.

In the cylinder hole of the protector 66, the biasingmember 67 is disposed to cover the periphery of the second column portion 77B of the support portion 77 and the periphery of the adjustment portion 72. One end portion of the biasing member 67 in the axial direction contacts the spring contact portion 88, and the other end portion of the biasing member 67 in the axial direction contacts the one end surface 77aA of the first column portion 77A. Moreover, the biasing member 67 biases the protector 66 toward the one side in the axial direction, i.e., toward the needle tip 21a of the needle tube 21.

Next, operation of the protector 66 of the injection needle assembly 60 according to the second embodiment having the above-described configuration will be described.

In the state before puncturing as illustrated in Fig. 5, the slide protrusion 87 is disposed on one side of the first slide portion 78a in the axial direction at the slide groove 78, and contacts the first stopper 81. In this state, the protector 66 covers the periphery of the second column portion 77B, the periphery of the adjustment portion 72 through which the needle tube 21 penetrates, and the periphery of the needle tip 21a of the needle tube 21. Thus, accidental puncturing with the needle tip 21a of the needle tube 21 before the skin is punctured with the needle tip 21a of the needle tube 21 can be prevented. Moreover, the slide protrusion 87 contacts the first stopper 81. This can prevents the protector 66 from dropping out of the support portion 77 of the first member 63.

Next, a tip end surface 66a of the protector 66 is pressed onto the skin against biasing force of the biasing member 67. The slide protrusion 87 of the protector 66 slides along the first slide portion 78a. Then, the protector 66 moves along the axial direction of the support portion 77, i.e., the axial direction of the needle tube 21. Thus, a needle protruding surface 72a of the adjustment portion 72 and the needle tip 21a of the needle tube 21 protrude from the insertion hole 88a of the protector 66, and therefore, the needle tip 21a of the needle tube 21 is exposed through the tip end surface 66a.

As illustrated in Fig. 6, the slide protrusion 87 slides from the first slide portion 78a to the second slide portion 78b through the inclinedportion 78c. In this state, the inclined portion 78c is inclined with respect to the axial direction of the support portion 77. Thus, when the slide protrusion 87 moves at the inclined portion 78c, the protector 66 rotates, with a predetermined angle, about the center of the support portion 77 along the circumferential direction of the support portion 77, i.e., the circumferential direction of the needle tube 21. In this state, the biasing member 67 is elastically deformed and compressed between the spring contact portion 88 of the protector 66 and the one end surface 77aA of the first column portion 77A.

Then, the slide protrusion 87 moves to the other endportion of the second slide portion 78b in the axial direction after having passed through the inclined portion 78c. The groove depth of the second slide portion 78b is formed greater than those of the first slide portion 78a and the inclined portion 78c. Thus, the slide protrusion 87 having moved to the second slide portion 78b does not return to the inclined portion 78c and the first slide portion 78a again.

The protector 66 further rotates with the predetermined angle because of the inclined portion 78c provided at the slide groove 78, and therefore, the moving distance of the protector 66 in the axial direction of the support portion 77 can be further shortened. This can shorten the length of the protector 66 in the axial direction, leading to size reduction of the injection needle assembly 60.

In a case where the above-described configuration is applied to a hypodermic injection apparatus configured to puncture a layer below a skin upper layer portion with a needle tube, the length of the needle tube piercing the skin is longer than that of the injection needle assembly 60 configured to puncture the skin upper layer portion with the needle tube 21 according to the present embodiment, and therefore, the length of the protector 66 in the axial direction is also increased. Thus, the rotating distance of a protector along the circumferential direction of a support portion is longer than that of the injection needle assembly 60 of the present embodiment. That is, in the hypodermic injection apparatus, the protector needs to greatly rotate on the skin, leading to the probability that a feeling of discomfort is provided to a user due to winding of the skin around the protector.

On the other hand, the injection needle assembly 60 of the present embodiment is for puncturing the skin upper layer portion with the needle tip 21a of the needle tube 21, and the protruding length L of the needle tube 21 is an extremely-short length of 0.5 to 3.0 mm. Thus, the length of the protector 66 in the axial direction can be also shortened, and the rotating distance of the protector 66 along the circumferential direction of the support portion 77 can be also extremely shortened. This can reduce, as a result, a feeling of discomfort provided to the user even when the protector 66 rotates upon puncturing of the skin with the needle tip 21a of the needle tube 21.

Next, upon separation from the tip end surface 66a of the protector 66, the biasing member 67 is released from pressing from the skin through the protector 66. Then, the protector 66 is biased toward the needle tip 21a of the needle tube 21 by restorative force (the biasing force) of the biasing member 67.

As illustrated in Fig. 7, the slide protrusion 87 slides in the second slide portion 78b, and moves over the return restriction portion 83 to contact the second stopper 82. Thus, movement of the protector 66 in the axial direction is restricted. Then, the protector 66 covers the periphery of the needle tip 21a of the needle tube 21, and the needle tube 21 is housed in the protector 66.

One end surface of the return restriction portion 83 in the axial direction stands substantially perpendicularly from the bottom surface of the second slide portion 78b. Thus, movement of the slide protrusion 87 from the one side to the other side in the axial direction of the second slide portion 78b is restricted. Thus, movement of the protector 66 from the one side to the other side in the axial direction after puncturing is restricted. This can prevent, after puncturing, the needle tip 21a of the needle tube 21 from protruding from the tip end surface 26a of the protector 66 again. As a result, the needle tip 21a of the needle tube 21 after use can be held in a safe state, and puncturing with the needle tip 21a of the needle tube 21 after use despite user's intention can be prevented.

Other configurations are similar to those of the injection needle assembly 2 of the first embodiment, and therefore, description thereof will not be repeated. Features and advantageous effects similar to those of the injection needle assembly 2 of the first embodiment as described above can be also obtained by the injection needle assembly 60 having the above-described configuration.

In the injection needle assembly 60 of the second embodiment, the biasing member 67 is also housed in the protector 66, and is also disposed at a position apart from the slide groove 78. This can prevent contact among the members when the biasing member 67 elastically deforms or when the slide protrusion 87 slides at the slide groove 78. As a result, movement and rotation operation of the protector 66 can be smoothly performed.

In the above-described second embodiment, the example where the slide protrusion 87 is provided at the protector 66 and the slide groove 78 is provided at the support portion 77 has been described, but the present invention is not limited to such an example. The slide protrusion 87 may be provided at the support portion 77, and the slide groove 78 may be provided at the protector 66.

Further, in the above-described second embodiment, the length of extension of the second slide portion 78b in the axial direction is set longer than that of the first slide portion 78a, and the groove depth of the second slide portion 78b is set greater than those of the first slide portion 78a and the inclined portion 78c. However, the present invention is not limited to such a configuration.

For example, the length of extension of the second slide portion 78b in the axial direction is set shorter than that of the first slide portion 78a. Thus, the inclined portion 78c is inclined from the other side to the one side in the axial direction of the support portion 77 while extending from the first slide portion 78a to the second slide portion 78b along the circumferential direction of the support portion 77. Moreover, the groove depth of the inclined portion 78c is set greater than that of the first slide portion 78a, and is set identical to that of the second slide portion 78b. Further, the groove depth of the end portion of the first slide portion 78a close to the inclined portion 78c is set identical to that of the inclined portion 78c.

According to this configuration, when the protector 66 is pressed against the skin, the slide protrusion 87 slides at the first slide portion 78a, and moves along the axial direction of the support portion 77. Then, the slide protrusion 87 moves to the end portion of the first slide portion 78a close to the inclined portion 78c. When the protector 66 is separated from the skin, the slide protrusion 87 slides at the inclined portion 78c and the second slide portion 78b. Since the groove depth of the end portion of the first slide portion 78a close to the inclined portion 78c is set greater than those of other portions of the first slide portion 78a, the slide protrusion 87 slides at the inclined portion 78c without sliding at the first slide portion 78a again.

Moreover, the slide protrusion 87 slides at the inclined portion 78c, and accordingly, the protector 66 rotates along the circumferential direction of the support portion 77. Thus, the protector 66 rotates when separated from the skin. This can reduce a feeling of discomfort provided to the user due to rotation of the protector 66 on the skin.

The embodiments of the medicine injection apparatus and the injection needle assembly according to the present invention have been, including the features and the advantageous effects thereof, described above. However, the medicine injection apparatus and the injection needle assembly according to the present invention are not limited to the above-described embodiments, and variations can be made without departing from the gist of the present invention described in the claims.

In the above-described embodiments, the example where the lure lock portion is provided as the lock mechanism 12 has been described, but the present invention is not limited to such an example. For threadedly-engagement, the external thread port ion may be provided at the discharge portion, and the internal thread portion may be provided at the cylinder hole of the second member 24 of the injection needle assembly 2.

### Reference Signs List

1 ... medicine injection apparatus, 2, 60 ... injection needle assembly, 3 ... syringe, 4 ... pusher member, 21 ... needle tube, 21a ... needle tip, 22, 62 ... needle hub, 23, 63 ... first member, 24, 64 ... second member, 26, 66 ... protector, 26a, 66a ... tip end surface, 27, 67 ... biasing member, 28 ... restriction mechanism, 29 ... contact piece, 29a ... inclined surface, 30, 88 ... spring contact portion, 30a ... insertion hole, 31, 71 ... base portion, 32, 72 ... adjustment portion, 32a, 72a ... needle protruding surface, 33, 73 ... stabilization portion, 33a ... end surface, 34, 74 ... guide portion, 34a ... contact surface, 36, 76 ... housing recessed portion, 36a, 76a ... bottom surface, 37 ... support portion, 37A, 77A ... first column portion, 37aA, 37aB, 77aA, 77aB ... one end surface, 37B, 77B ... second column portion, 40, 80 ... space, 41 ... lock member, 42 ... expansion member, 43 ... retaining member, 43a ... support hole, 45 ... cylindrical portion, 46 ... lock portion, 46a ... lock piece, 48 ... expansion portion, 48a ... expansion surface, 78 ... slide groove, 78a ... first slide portion, 78b ... second slide portion, 78c ... inclined portion, 81 ... first stopper, 82 ... second stopper, 83 ... return restriction portion, 87 ... slide protrusion, 88a ... insertion hole

## Claims

1. An injection needle assembly comprising:
a needle tube having a needle tip with which a biological body is able to be punctured;
a needle hub configured to hold the needle tube;
a protector movable between a first position at which the needle tip of the needle tube is covered and a second position at which the needle tip of the needle tube is exposed;
a biasing member configured to bias the protector toward the needle tip of the needle tube along an axial direction of the needle tube; and
a restriction mechanism configured to restrict the protector from moving from the first position to the second position again after the protector has moved from the first position to the second position and has further moved from the second position to the first position,
wherein the biasing member is disposed close to the needle tip of the needle tube in the axial direction with respect to the restriction mechanism.

2. The injection needle assembly according to claim 1, wherein
one of the needle hub or the protector is provided with a slide groove,
the remaining one of the needle hub or the protector is provided with a slide protrusion slidably inserted into the slide groove, and
the restriction mechanism includes the slide protrusion and a return restriction portion provided at the slide groove to contact the slide protrusion.

3. The injection needle assembly according to claim 1, wherein
the restriction mechanism includes:
a lock member having a lock portion elastically deformable outward in a radial direction of the needle tube, and
an expansion member detachably attached to the protector and configured to expand, outward in the radial direction, an opening formed by the lock portion when the protector moves from the first position to the second position,
when the protector moves from the first position to the second position, the expansion member is, together with a contact piece provided with the protector, inserted into the lock member through the lock portion, and
when the protector returns from the second position to the first position, the expansion member is detached from the protector, and is locked at the lock portion.

4. The injection needle assembly according to any one of claims 1 to 3, further comprising:
an adjustment portion
provided at a periphery of the needle tube, and
having a needle protruding surface which is configured to contact a skin when the biological body is punctured with the needle tube and from which the needle tip of the needle tube protrudes,
wherein the protector covers a periphery of the adjustment portion at the first position.

5. The injection needle assembly according to any one of claims 1 to 4, further comprising:
a stabilization portion
extending from the needle hub,
disposed to cover the periphery of the needle tube and a periphery of the protector, and
having an end surface configured to contact the skin when the biological body is punctured with the needle tube; and
a guide portion formed in a flange shape protruding substantially perpendicularly from an outer peripheral surface of the stabilization portion toward an outside in the radial direction.

6. A medicine injection apparatus comprising:
an injection needle assembly including a needle tube having a needle tip with which a biological body is able to be punctured; and
a syringe detachably attached to the injection needle assembly,
wherein the injection needle assembly includes:
a needle hub configured to hold the needle tube,
a protector movable between a first position at which the needle tip of the needle tube is covered and a second position at which the needle tip of the needle tube is exposed,
a biasing member configured to bias the protector toward the needle tip of the needle tube along an axial direction of the needle tube, and
a restriction mechanism configured to restrict the protector from moving from the first position to the second position again after the protector has moved from the first position to the second position and has further moved from the second position to the first position, and
the biasing member is disposed close to the needle tip of the needle tube in the axial direction with respect to the restriction mechanism.
